# EUROPEAN PATENT APPLICATION

(11) **EP 1 955 711 A1**
(43) Date of publication of application: **13.08.2008**
(21) Application number: 07002779.2
(22) Date of filing: 09.02.2007
(51) Int. Cl.: A61K 47/48

(54) **Composition comprising covalent conjugates of chitosan and an acidic drug for parenteral administration**

(71) Applicant: The Jordanian Pharmaceutical Manufacturing Co., 11710 Naor (JO)
(72) Inventor: Badwan Adnan. Dr, 11185 Amman (JO); AhmadAl-Remawi Mayyas Mohammad, 13713 Russeifa (JO)
(74) Representative: Winkler, Andreas Fritz Ernst

(57) **Abstract**

The present invention relates to a composition comprising an aqueous solvent and a conjugate of at least one hydrophilic polymer having a primary amine functional group, a derivative or monomer thereof and at least one acidic drug; a method for its preparation and use thereof

## Description

The present invention relates to an aqueous composition comprising an acidic drug and a hydrophilic polymer having a primary amine functional group.

Controlled release systems include any drug delivery system that achieves slow release of drug i.e. releases the drug over an extended period of time. Controlled release systems can be divided based on the route of administration into:
*Topical controlled release:* defined as a slow drug release after administration of a pharmaceutical dosage form where the desired effect is local, the dosage form is applied directly where its action is desired.
*Enteral controlled release:* defined as a slow release of a pharmaceutical dosage form where the desired effect is systemic (non-local), the dosage form is given via the digestive tract.
*Parenteral controlled release:* defined as a slow release of a pharmaceutical dosage form where the desired effect is systemic, and the dosage form is given by other routes than the digestive tract or alimentary canal, including intravenous, intramuscular, subcutaneous, intraocular, surgical implantation, etc. The following patents are examples about sustained release formulations for parenteral administration; W02005051418, WO2005051418, W00189479, W09824504 and EP1374860.

However, the delivery of a sustained release formulation directly to the blood stream via intravenous route is considered a big challenge. Most parenteral controlled release preparations are composed of micro-particle systems that are usually given intramuscularly or subcutaneously. The micro-particles slowly erode with time and allow the release of an active material over an extended period of time.

Solution dosage forms containing soluble excipients and drugs are usually suitable for intravenous administration. Poorly soluble drugs or excipients are usually not suitable to be administered intravenously.

It is an object of the present invention to provide a pharmaceutical composition which allows parenteral administration of substantially water-insoluble drugs, preferably in solution dosage form. Additionally, a method for its preparation and use thereof shall be provided.

The first object is achieved by a composition comprising an aqueous solvent and a conjugate of at least one hydrophilic polymer having a primary amine functional group, a derivative or monomer thereof and at least one acidic drug.

Preferably, the polymer is chitosan or the monomer is glucosamine.

In this context is also preferred that the acidic drug contains an acidic functional group selected from the group consisting of carboxylic, sulfonic, nitric, phosphoric group, or a salt thereof.

Most preferably, the acidic drug is insoluble in water.

The acidic drug may be selected from beta lactam antibiotics, quinolone antibiotics, sulfonamide, nonsteroidal anti-inflammatory drugs, anti-hyperlipidimic drugs, psychostimulants, prostaglandin vasodilators, antidepressants, anticonvulsive agents, hemostatic agents, anti-tuberculosis agents, hepatic protectant agents, flavoring agents, sweeteners, antispasmodic agents, anti-neoplastic agents, antiseptic agents, anti-inflammatory cortisone drugs, hypoglycemic drugs, anti-arrythmetic agents, antihypertensive agents, anti-allergy agents, emollients, agents for gastrointestinal disorders and Anti-infective.

In this regard, the acidic drug may be selected from cephalosporins, penicillins, nalidixic acids, ciprofloxacin, acediasulfone, amfenac, diclofenac, ibuprofen, indomethacin, acetyl salicylic acid, clinadac, naproxen, mefenamic acid, fosfomycin, bendazac, acifran, fluvastatin, atorvastatin, aceglutamide, alprostadil, amineptine, gamma aminobutyric acid, gabapentin, valproic acid, aminocaproic acid, aminosalicylic acid, amino acid arginine, aspartic acid, betaine, aspartam, baclofen, bendamustine, bromebric acid, sodium borocaptate, chlorambucil, methotrexate, bismuth subgallate, dodicin, betamethasone, calcium mesoxalate, capobenic acid, captopril, cromolyn sodium, spaglumic acid, docusate sodium, mesalamine and flumequine.

The composition is preferably characterized in that the aqueous solvent has a pH of 4 to 7, preferably 4 to 6.5.

The aqueous solvent may be 0.01 M HCl solution.

Preferably, the chitosan has a weight average molecular weight ranging from 20.000 to 1.000 Da.

The composition may further comprise at least one additional therapeutically active ingredient other than the acidic drug as disclosed above.

Preferably, the other therapeutically active ingredient is immediately released after oral administration and the acidic drug in the conjugate has a controlled release characteristic.

Also it is preferred that the composition additionally comprises pharmaceutical excipients selected from the group consisting of sweeteners, coloring agents, preservatives, thickeners and flavoring agents.

According to the invention is also a method for preparing a conjugate of at least one hydrophilic polymer having a primary amine functional group, a derivative or monomer thereof and at least one acidic drug, comprising the steps of reacting the acidic drug to obtain an acid halide, ester, anhydride or other intermediate product and reacting the intermediate product with the hydrophilic polymer to form a conjugate via amide bond formation.

Preferably, the method is carried out under acidic environment.

The composition may be used in the preparation of a medicament for parenteral administration, preferably this parenteral administration is intravenous, intramuscular, subcutaneous, intraocular or by surgical implantation.

Surprisingly, it was found that according to the present invention a composition is provided wherein an as such insoluble acidic drug can be transferred into a soluble form by forming a conjugate of this drug with a hydrophilic polymer having a primary amine functional group. Thus, according to the present invention, a water-insoluble drug is conjugated to a highly soluble and biodegradable, hydrophilic polymer. The drug-polymer-conjugate becomes water-soluble. This allows the direct delivery of the drug to the blood stream via intravenous route as a novel parenteral controlled release aqueous solution. Preferably, chitosan oligosaccharide having an weight average molecular weight of 20.000 to 1.000 Da is used in this invention as a highly water-soluble biodegradable polymer. The conjugate of polymer and acidic drug is dissociated slowly inside the human body via the liver or blood enzymes and results in the formation of free drug molecules. These free molecules are now available for their pharmacological action.

Chitosan (poly(N-acetylglycosamine)) is partially de-acetylated chitin which is one of the most abundant polysaccharides in nature second only to cellulose. It has a sugar backbone consisting of β-1,4 linked glucosamine with a high degree of N-acetylation (70-90% N-acetylglucosamine and 10-30% D-glucose units), a structure very similar to that of cellulose; the only difference being the replacement of the hydroxyl by amino groups.

Chitosan use in drug delivery system is well known. S.T.P. Pharma in Jan-Feb 2000 published a complete thematic issue on "chitosan in drug delivery systems". Also, there are several review articles on chitosan as a drug carrier.

[W. Paul, C. Sharma. Chitosan, a drug carrier for the 21st century: a review. STP Pharma Sci.,10, pp 5-22, 2000; F. Olivia, P. Buri., R. Gury. Chitosan: A unique Polysaccharide for Drug Delivery: a review. Drug Dev. Ind.Pharm. 24, pp 979-993, 1998].

Ibuprofen represents a good model drug for conjugation with chitosan polymer. Ibuprofen is non-steroidal anti-inflammatory product. The molecular weight is 206 g/mole. It is practically insoluble in water. Thus, there is no ibuprofen IV solutions. Ibuprofen is rapidly absorbed from GIT after oral administration. Peak plasma levels of 15 to 20 mcg/ml occur about 1 hr after administration of a single 200-mg oral dose in fasting subjects. The serum half-life after a single 200 mg oral dose in fasting subjects was about 1.9 hr.

Chitosan has a wide range of molecular weights. Chitosan of large molecular weight of more than 50.000 Da (weight average molecular weight) has slow water solubility and forms high viscosity solutions in acidic aqueous medium. Low molecular weight chitosans of less than 50.000 Da are usually water-soluble and form low viscosity solutions. Chitosan having molecular weight ranging from 20.000 to 1.000 Da may refer to as chitosan oligosaccharides. Chitosan oligosaccharides are the most preferred ones for the present invention.

Especially, chitosan oligosaccharides are known for their high solubility. The conjugation of an acidic drug such as ibuprofen can be easily achieved by different chemical reaction. This can be achieved via conversion of ibuprofen to a suitable activated intermediate that can react with chitosan primary amine groups. This intermediate can be achieved by forming an acid halide, ester, anhydride or any activation process known in the art. Upon reacting activated intermediate with chitosan a drug chitosan conjugate is formed. The degree of conjugation is controlled to produce a water-soluble drug chitosan conjugate. The drug was detected in the plasma of rabbits upon giving the conjugate in a form of intravenous solution. The mechanism of drug release from the solution is not well established. The conjugate might release the active material upon hydrolyzing the conjugate via the liver enzymes or lysozymes.

Additional features and advantages of the subject-matter of the present invention can be taken from the following detailed description thereof with reference to the accompanying drawings, wherein
Fig. 1 illustrates the formation of a conjugate comprising chitosan and ibuprofen;
Fig. 2 shows a structure of a water-soluble chitosan-acidic drug conjugate system;
Fig. 3 shows UV-spectra of ibuprofen (A) and ibuprofen ethanoate (B);
Fig. 4 shows IR spectra of chitosan (A) and chitosan ibuprofen conjugate (B); and
Fig. 5 shows plasma concentration time profil of ibuprofen when ibuprofen chitosan solution (amide conjugate) is given to rabbits via intravenous route in a dose of 3 mg/kg.

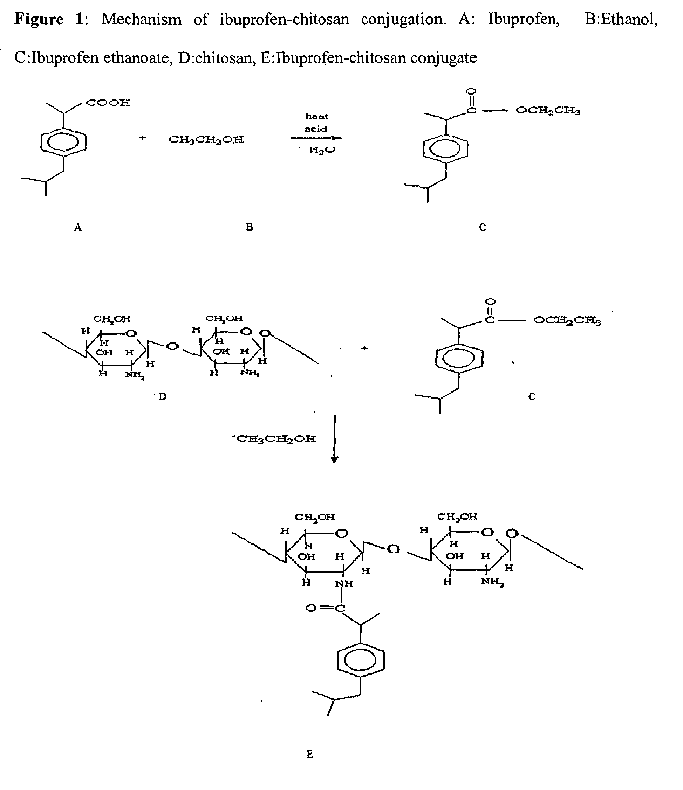

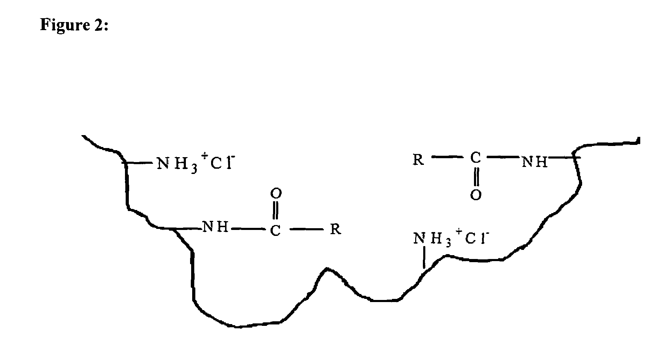

Chitosan-ibuprofen conjugate results by the formation of a covalent amide bond, Fig. 1. The amide bond is formed by chemical interaction during solution formulation. Where, ethanol acts as an intermediate for the formation of the amide link, i.e. it helps in the formation of an ibuprofen ester that interacts with primary amine groups in chitosan polymer to form the amide bond and leaves the solution as a by-product which is get rid of evaporation. Acidic environment aids ibuprofen ester formation. In addition, it helps in acidification of free primary amine groups in chitosan (protonation) and solubilization in aqueous medium. Ibuprofen ethanoate, water-insoluble ester, is mixed with chitosan aqueous solution and forms an emulsion. The end point of conjugation is obtained once homogenous clear solution is obtained. The incomplete amide bond formation between the drug and chitosan is expected. The free primary amine groups are useful to be used as a source for ionization of the free primary amine groups in the chitosan-drug conjugate when placed in acidic conditions, Fig. 2. This would increase total polymer solubility. A clear solution is to be formed containing the polymer linked with the active material.

Examples on the acidic active material related to the invention are beta-lactam antibiotics such as cephalosporins and penicillins. Quinolone antibiotics such as nalidixic acid, ciprofloxacin. Sulfonamide such as acediasulfone. Nonsteroidal anti-inflammatory drugs such as amfenac, diclofenac, ibuprofen, acetyl salicylic acid, clinadac, naproxen, mefenamic acid, fosfomycin, bendazac. Antihyperlipidimic such as acifran, fluvastatin, atorvastatin. Psy-chostimulant such as aceglutamide. Prostaglandin vasodilator such as alprostadil. Antidepressant such as amineptine, gamma aminobutyric acid, gabapentin. Anticonvulsive agent such as valproic acid. Hemostatic agents such as aminocaproic acid. Anti-tuberculosis such as aminosalicylic acid. Hepatic protectant amino acid arginine, aspartic acid, betaine. Flavoring agents and sweeteners such as aspartam. Antispasmodic agent such as baclofen. Antineoplastic agent such as bendamustine, bromebric acid, sodium borocaptate, chlorambucil, methotrexate. Antiseptic such as bismuth subgallate, dodicin. Anti-inflammatory cortisone drugs such as betamethasone. Hypoglycemic oral drugs such as calcium mesoxalate. Antiarrythmetic agents such as capobenic acid. Antihypertesive agents such as captopril. Anti-allergy agents such as cromolyn sodium, spaglumic acid. Emollient such as docusate sodium. GI disorders such as mesalamine. Anti-infective such as flumequine

The water soluble conjugate when given parenterally is dissociated slowly and provides a sustained release behavior inside the human body via the liver or blood enzymes and results in formation of free drug molecules. These free molecules are now available for their pharmacological action.

### Examples

In all examples the chitosan used was purified and activated by washing of chitosan with n-hexane and then ethanol 96% until a clear spectrum in ethanol is obtained by UV spectrum. Then the purified chitosan was completely dried in oven before use to get rid of organic solvents.

### Example 1

### Conjugation reaction

Ibuprofen (carboxylic acid) was esterified using alcohol (ethanol). Ibuprofen ethanoate (insoluble) was reacted with purified chitosan oligosaccharide with weight average molecular weight <3000 in an aqueous solution to form a homogenous solution containing ibuprofen-chitosan conjugate, **Fig 1****.** Slight acidic medium of hydrochloric acid may enhance the conjugation process and increase solubility of ibuprofen chitosan conjugate in aqueous solution, **Fig 2****.**

### Example 2

### Characterization of conjugation

Infrared spectra were obtained using Fourier transform infra red spectrometer (FTIR). FTIR was performed under room air at room temperature and KBr disk. Samples were placed in oven at 105°C for 3 hrs before doing any measurements to get rid of moisture.

Approximately 500 mg of KBr and 5 mg of sample powder were blended with pestle and mortar for 5 min.The sample disk was prepared at a pressure of 9 tons for 21 min.

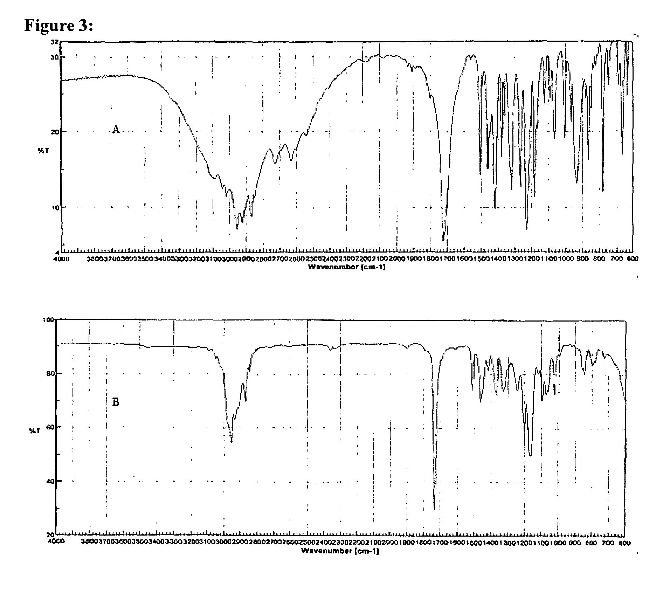

The infrared spectra of ibuprofen and its ethanoate ester are shown in **Fig 3****.** The spectra differences indicate that there may be a slight difference in the chemical structure. In case of infrared spectra, the large peaks width between 2850-3500 cm-1, which corresponds to hydrogen bonding in ibuprofen carboxylic acid functional groups, decreased tremendously indicating the transformation of the carboxylic acid to ethyl carboxylate of ibuprofen ethanoate ester. A shift in carbonyl functional group for ibuprofen of wave number 1720 to 1740 cm-1 indicates the formation of ibuprofen ethanoate.

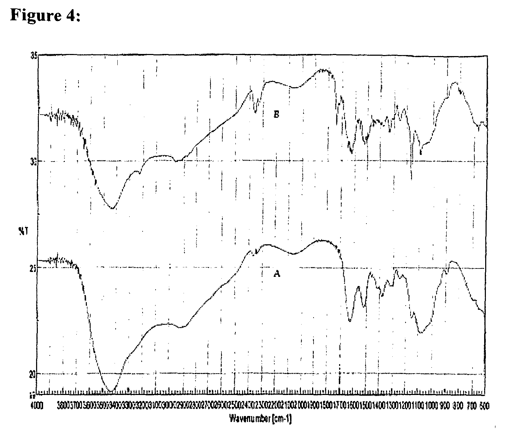

The amide bond formation between ibuprofen and chitosan was indicated by physical conversion of the emulsion into a clear transparent solution. In addition, amide bond formation was indicated by the appearance of the carbonyl band at 1740 cm-1 and the C-O band at 1160 cm-1, as shown in **Fig 4****.** Where, these bands were not observed in the IR spectrum of chitosan.

### Example 3

### In vivo evaluation of drug release

Ibuprofen was conjugated to chitosan having weight average molecular weight of less than 3.000 as described in Example 1, where a clear solution was obtained for controlled release delivery.

The conjugate containing ibuprofen and chitosan solution was given intravenously to rabbits in a dose of 3 mg of ibuprofen/kg. Blood samples were withdrawn at specified time interval, centrifuged at 3500 rpm for 10 minutes and placed in a freezer at -20 °C . The samples were analyzed using HPLC method. A simple rapid method of determining the ibuprofen concentration by HPLC was developed. Naproxen was used as an internal standard. Mobile Phase: Water: Acetonitrile (40:60), then adjust pH to 2.4 with H3PO4. Column: Waters, Symmetry, 5u, C18, and 150*4.6 mm. Internal STD Stock Solution: Dissolve 5 mg of Naproxen into 50 ml Methanol. Injection Loop: 50ul. Flow rate: 1 ml/min. Wavelength: 220 nm.

Sample Preparation: Transfer 100 microliter of plasma sample to test tube, Add 10 microliter of Internal STD Stock solution, Add 0.25 ml of 1 M HCl, Shake for 30 seconds, Add 5 mL of (85:15)(Hexane: Isopropanol) Shake with vortex for 1 min., Centrifuge at 3000 rpm for 10 min., Transfer 4 ml of organic layer to new test tube, Evaporate the organic solvent using an air shower, and Reconstitute with 1 ml mobile phase. The method was evaluated for specificity showing that there is no interference with the ibuprofen peak. Recovery was 85-90% for ibuprofen. The calibration curve was linear over the concentration 0.5-10 microgram/ml.

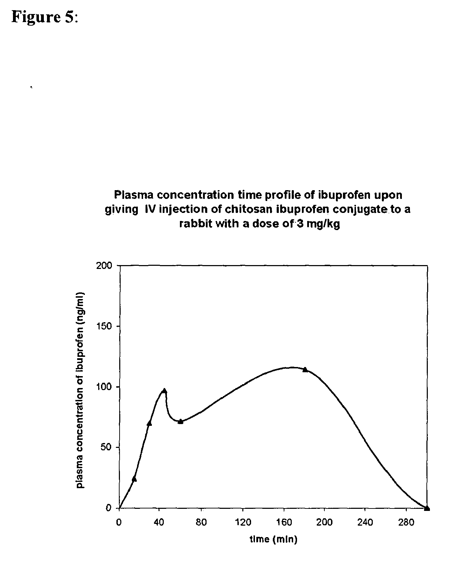

Controlled release behavior for the formula of ibuprofen conjugated with chitosan was confirmed for intravenous administration, Fig 5.

Above results illustrate that ibuprofen shows a zero order drug release upon dissociated from the conjugate in the rabbit blood.

The features disclosed in the foregoing description, in the claims and in the drawings may, both separately and in any combination thereof, be material for realizing the invention in diverse forms thereof.

## Claims

1. Composition comprising an aqueous solvent and a conjugate of at least one hydrophilic polymer having a primary amine functional group, a derivative or monomer thereof and at least one acidic drug.

2. Composition according to claim 1, wherein the polymer is chitosan or the monomer is glucosamine.

3. Composition according to claim 1 or 2, wherein the acidic drug contains an acidic functional group selected from the group consisting of carboxylic, sulfonic, nitric, phosphoric group, or a salt thereof.

4. Composition according to any of the preceding claims, wherein the acidic drug is insoluble in water.

5. Composition according to claim 1, wherein the acidic drug is selected from beta lactam antibiotics, quinolone antibiotics, sulfonamide, nonsteroidal anti-inflammatory drugs, antihyperlipidimic drugs, psychostimulants, prostaglandin vasodilators, antidepressants, anticonvulsive agents, hemostatic agents, anti-tuberculosis agents, hepatic protectant agents, flavoring agents, sweeteners, antispasmodic agents, anti-neoplastic agents, antiseptic agents, anti-inflammatory cortisone drugs, hypoglycemic drugs, antiarrythmetic agents, antihypertensive agents, anti-allergy agents, emollients, agents for gastrointestinal disorders and anti-infective.

6. Composition according to claim 5, wherein the acidic drug is selected from cephalosporins, penicillins, nalidixic acids, ciprofloxacin, acediasulfone, amfenac, diclofenac, ibuprofen, indomethacin, acetyl salicylic acid, clinadac, naproxen, mefenamic acid, fosfomycin, bendazac, acifran, fluvastatin, atorvastatin, aceglutamide, alprostadil, amineptine, gamma aminobutyric acid, gabapentin, valproic acid, aminocaproic acid, aminosalicylic acid, amino acid arginine, aspartic acid, betaine, aspartam, baclofen, bendamustine, bromebric acid, sodium borocaptate, chlorambucil, methotrexate, bismuth subgallate, dodicin, betamethasone, calcium mesoxalate, capobenic acid, captopril, cromolyn sodium, spaglumic acid, docusate sodium, mesalamine and flumequine.

7. Composition according to any of the preceding claims, wherein the aqueous solvent has a pH of 4 to 7, preferably 4 to 6.5.

8. Composition according to claim 7, wherein the aqueous solvent is 0.01 M HCl solution.

9. Composition according to any of the preceding claims, wherein the chitosan has a weight average molecular weight of 20.000 to 1.000 Da.

10. Composition according to any of the preceding claims, further comprising at least one additional therapeutically active ingredient other than the acidic drug as disclosed in claim 1.

11. Composition according to claim 10, wherein the additional therapeutically active ingredient is immediately released after administration, and the acidic drug has controlled release characteristic.

12. Composition according to any of the preceding claims, additionally comprising pharmaceutical excipients selected from the group consisting of sweeteners, coloring agents, preservatives, thickeners and flavoring agents.

13. Method for preparing a conjugate of at least one hydrophilic polymer having a primary amine functional group, a derivative or monomer thereof and at least one acidic drug, comprising the steps of reacting the acidic drug to obtain an acid halide, ester, anhydride or other intermediate product and reacting the intermediate product with the hydrophilic polymer to form a conjugate via amide bond formation.

14. Method according to claim 13, wherein the method is carried out under acidic environment.

15. Use of the composition according to any of the claims 1 to 12 for preparing a medicament for parenteral administration.

16. Use according to claim 15, wherein parenteral administration is intravenous, intramuscular, subcutaneous, intraocular or by surgical implantation.
